# FASCICULE DE BREVET EUROPEEN

(11) **EP 0 988 076 B1**
(45) Date de publication et mention de la délivrance du brevet: **18.12.2002**
(21) Numéro de dépôt: 98929526.6
(22) Date de dépôt: 09.06.1998
(51) Int. Cl.: A61M 5/50, A61M 5/32

(54) **SERINGUE A AIGUILLE AUTO-ESCAMOTABLE**
SELBST-ZURÜCKZIEHENDE SPRITZEN-NADEL
SELF-RETRACTING SYRINGE NEEDLE

(30) Priorité: 10.06.1997 FR 9707185
(43) Date de publication de la demande: 29.03.2000
(73) Titulaire: BLUE STAR CORPORATION, Montevideo (UY); Bonnet, Jean Pierre, 77164 Ferrières (FR)
(72) Inventeur: BONNET, Jean, Pierre, F-77164 Ferrières (FR)
(74) Mandataire: Somnier, Jean-Louis
(86) Numéro de dépôt international: FR9801177
(87) Numéro de publication internationale: WO98056442

(56) Documents cités:
- WO-A-91/13643
- WO-A-92/09319
- WO-A-93/00949
- WO-A-95/23004
- CH-A- 685 979
- FR-A- 2 680 110

## Description

La présente invention concerne une seringue à aiguille auto-escamotable.

Elle concerne également un circlips de blocage en translation pour deux corps coulissant l'un dans l'autre.

Les seringues médicales servent à injecter à l'aide d'une aiguille creuse une substance, généralement médicamenteuse, dans le corps du patient.

L'emploi s'est maintenant généralisé de seringues dites à usage unique, vendues stériles dans un emballage clos et ne devant pas être réutilisées, ceci afin d'éviter tout risque de contamination accidentelle d'un patient à l'autre. Mais ce matériel, largement répandu, ne protège pas le personnel médical d'une piqûre accidentelle et n'interdit pas matériellement le réemploi de la seringue si l'usager ne procède pas à sa destruction volontaire après emploi.

Le document PCT/FR90/00572 décrit une seringue à aiguille auto-escamotable, comprenant un corps portant à une extrémité des moyens de support d'une aiguille d'injection, un piston mobile dans le corps, un fourreau à l'intérieur duquel est susceptible de coulisser le corps, et un ressort disposé entre le fourreau et le corps. Il est aussi prévu au moins un premier ergot souple porté par le fourreau de manière à coopérer en butée avec un premier épaulement pour définir une première position de blocage du corps par rapport au fourreau dans un premier sens, le ressort étant comprimé et l'aiguille sortant du fourreau dans cette première position, et avec un second épaulement pour définir une seconde position de blocage dans le même sens, le ressort étant au moins partiellement débandé et l'aiguille étant rentrée dans le fourreau dans cette seconde position. Le passage de la première position à la seconde position se fait par la coopération du piston avec l'ergot souple pour libérer en fin de course du piston l'ergot du premier épaulement et provoquer ainsi, sous l'effet du ressort, le recul du corps dans le fourreau jusqu'à son blocage en seconde position.

Cette seringue comprend au moins un second ergot souple et un troisième épaulement, de sens contraire respectivement au premier ergot souple et aux deux premiers épaulements, positionnés respectivement sur le fourreau et la paroi du corps pour bloquer le corps dans le fourreau, lorsqu'il est dans la seconde position de blocage, dans le sens inverse du premier sens.

Cependant, la seringue de type connu présente l'inconvénient d'avoir des ergots souples qui peuvent être facilement cassés lorsque l'utilisateur cherche à ramener le corps dans sa première position de blocage dans le fourreau en vue d'une nouvelle utilisation de la seringue.

Les ergots souples de blocage ne garantissent donc pas, de manière certaine, une utilisation unique de la seringue.

On connaît également dans le document FR 2 680 110 une seringue ayant un corps susceptible de coulisser à l'intérieur d'un fourreau et d'être bloqué en coulissement dans ce fourreau grâce à un circlips qui vient se loger dans deux gorges disposées en vis-à-vis dans la position de blocage, une des gorges étant formée sur la paroi extérieure du corps de la seringue et l'autre gorge étant formée sur la paroi intérieure du fourreau.

Ce système de blocage est compliqué à mettre en oeuvre car il nécessite de former des gorges à l'intérieur du fourreau et à l'extérieur du corps de seringue.

La présente invention a pour but de proposer une seringue à usage unique et aiguille auto-escamotable, empêchant l'utilisateur de ressortir l'aiguille du fourreau après l'utilisation de la seringue.

La seringue à aiguille auto-escamotable visée par l'invention comprend un corps portant à une extrémité des moyens de support d'aiguille d'injection, un piston mobile dans le corps, un fourreau à l'intérieur duquel est susceptible de coulisser ledit corps, le fourreau comportant à une extrémité une partie formant manchon, un ressort disposé entre le fourreau et le corps, au moins un ergot souple coopérant en butée avec une portion dudit corps pour définir une première position de blocage du corps par rapport au fourreau dans un premier sens, le ressort étant comprimé et l'aiguille sortie du fourreau dans cette première position, et des moyens de blocage pour bloquer le corps dans le fourreau dans une seconde position de blocage, le ressort étant au moins partiellement débandé et l'aiguille étant rentrée dans le fourreau dans cette seconde position, le passage de la première position à la seconde position se faisant par la coopération du piston avec ledit ergot souple pour libérer sensiblement en fin de course du piston, l'ergot de ladite portion du corps et provoquer ainsi sous l'effet du ressort le recul du corps dans le fourreau jusqu'à son blocage en seconde position.

Selon l'invention, cette seringue est caractérisée en ce que les moyens de blocage comprennent un circlips monté autour dudit corps dans une position comprimée entre le fourreau et le corps dans ladite première position de blocage et dans une position relâchée dans ladite seconde position de blocage, le circlips ayant au moins une partie logée à l'intérieur de la partie formant manchon dans ladite seconde position de blocage entre ladite extrémité du fourreau et au moins un épaulement de la partie formant manchon adapté à coopérer en butée avec le circlips dans ladite seconde position de blocage pour bloquer le corps dans le fourreau dans ledit premier sens.

Grâce au circlips qui se détend dans la position relâchée, toute nouvelle introduction du corps dans le fourreau est empêchée, évitant par là-même la sortie de l'aiguille.

La coopération du circlips en butée avec l'extrémité du fourreau procure un blocage en position du corps par rapport au fourreau résistant beaucoup mieux aux tentatives de réintroduction du corps dans le fourreau que les ergots souples, utilisés dans les seringues connues, qui forment des points de blocage ponctuels facilement déformables ou destructibles.

La partie formant manchon autour du fourreau forme un logement pour le circlips en position relâchée, de sorte que l'utilisateur ne peut pas accéder au circlips et tenter de le ramener en position comprimée pour le réintroduire dans le fourreau.

Selon une version préférée de l'invention, le circlips a une section transversale en forme de L, la jambe de la forme en L étant logée entre le fourreau et le corps dans la seconde position de blocage.

Cette disposition permet de bloquer tout mouvement relatif du fourreau et du corps de seringue à la fois dans la direction longitudinale de la seringue mais également dans un plan transversal de la seringue.

D'autres particularités et avantages de l'invention apparaîtront encore dans la description ci-après.

Aux dessins annexés, donnés à titre d'exemple non limitatifs:
- la figure 1 est une vue en coupe longitudinale d'une seringue conforme à l'invention;
- les figures 2 et 3 sont des vues analogues à la figure 1 illustrant le fonctionnement de la seringue;
- la figure 4 est une vue de dessous d'un circlips conforme à l'invention;
- la figure 5 est une.vue en coupe selon la ligne V-V à la figure 4;
- la figure 6 est une vue en coupe du corps de la seringue conforme à l'invention;
- la figure 7 est une vue de dessus de la partie formant manchon du fourreau de la seringue conforme à l'invention;
- la figure 8 est une vue en coupe selon la ligne VII-VII à la figure 7;
- la figure 9 est une vue en coupe selon la ligne IX-IX à la figure 7;
- les figures 10 et 11 sont des vues en coupe d'un circlips conforme à d'autres modes de réalisation;
- les figures 12, 13 et 14 sont des vues schématiques illustrant divers moyens de réalisation d'une gorge sur un corps de seringue conforme à l'invention;
- la figure 15 est une vue en coupe longitudinale d'une autre version de la seringue conforme à l'invention, en position de stockage ;
- la figure 16 est une vue en coupe montrant uniquement le corps et le piston de la seringue selon la figure 15 ;
- les figures 17 et 18 sont deux vues analogues à la figure 15 illustrant le fonctionnement de la seringue.

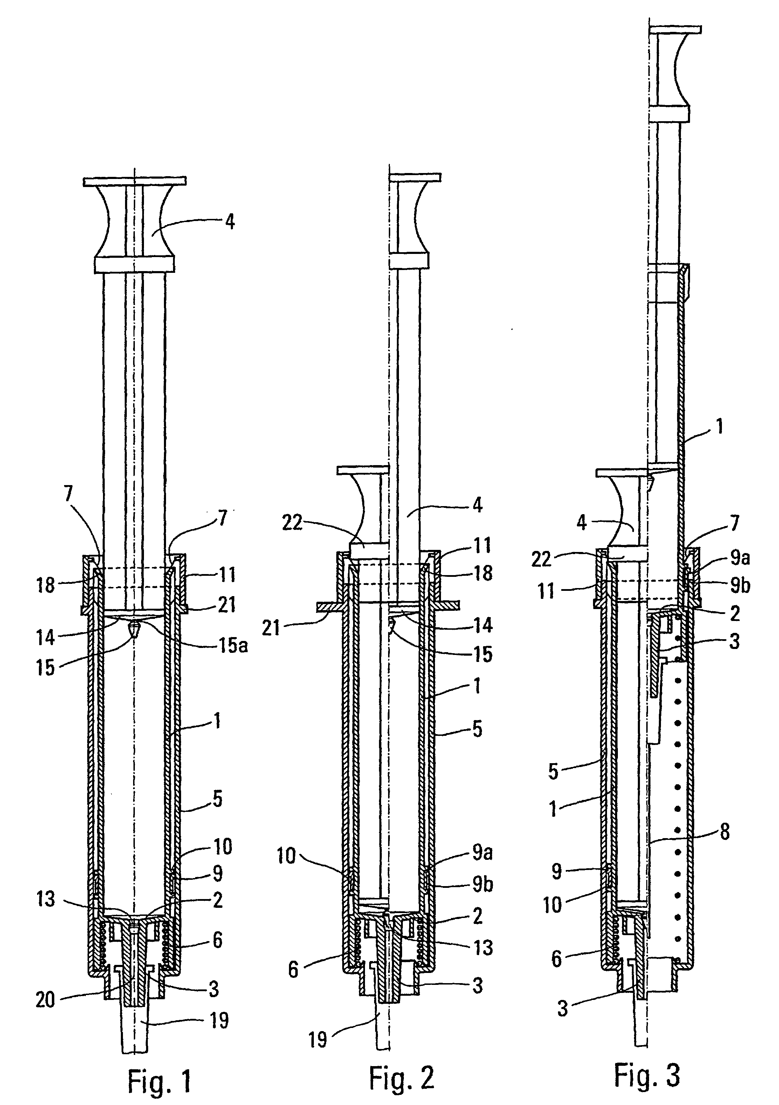

En référence à la figure 1 tout d'abord, la seringue à aiguille auto-escamotable comprend un corps 1 portant à une extrémité 2 des moyens de support d'aiguille d'injection 3, un piston 4 mobile dans le corps 1, un fourreau de protection 5 à l'intérieur duquel est susceptible de coulisser le corps 1 et un ressort 6 disposé entre le fourreau 5 et le corps 1.

Le corps 1 comporte une extrémité ouverte et une extrémité partiellement fermée 2.

Tenant au fond de l'extrémité 2, est formé un support d'aiguille 3 comportant une partie tronconique servant de téton de fixation d'une aiguille classique à embout de fixation tronconique 19. La partie tronconique du support d'aiguille 3 est percée d'un alésage 20 faisant communiquer l'intérieur du corps 1 avec l'alésage de l'aiguille.

Par l'extrémité ouverte du corps 1, peut pénétrer dans le corps 1 un piston 4 coulissant librement à l'intérieur du corps 1.

Le fourreau 5 sensiblement cylindrique est monté coulissant sur le corps 1. A une de ses extrémités, sa paroi se rétrécit pour former un orifice suffisant pour le passage du téton de fixation de l'aiguille, et dont le contour peut servir d'appui au ressort de compression 6 dont l'autre extrémité s'appuie sur un épaulement du corps 1, au niveau de l'extrémité 2.

Le fourreau 5 comporte, à son autre extrémité, une collerette 21 d'appui des doigts.

La seringue comporte en outre au moins un ergot souple 7 porté par le fourreau 5, de manière à coopérer en butée avec une portion 18 du corps 1 pour définir une première position de blocage du corps 1 par rapport au fourreau, dans un premier sens.

La figure 1 illustre la seringue dans cette première position de blocage, correspondant à la position de stockage de la seringue avant utilisation, le ressort 6 étant comprimé et l'aiguille 8 sortie du fourreau 5.

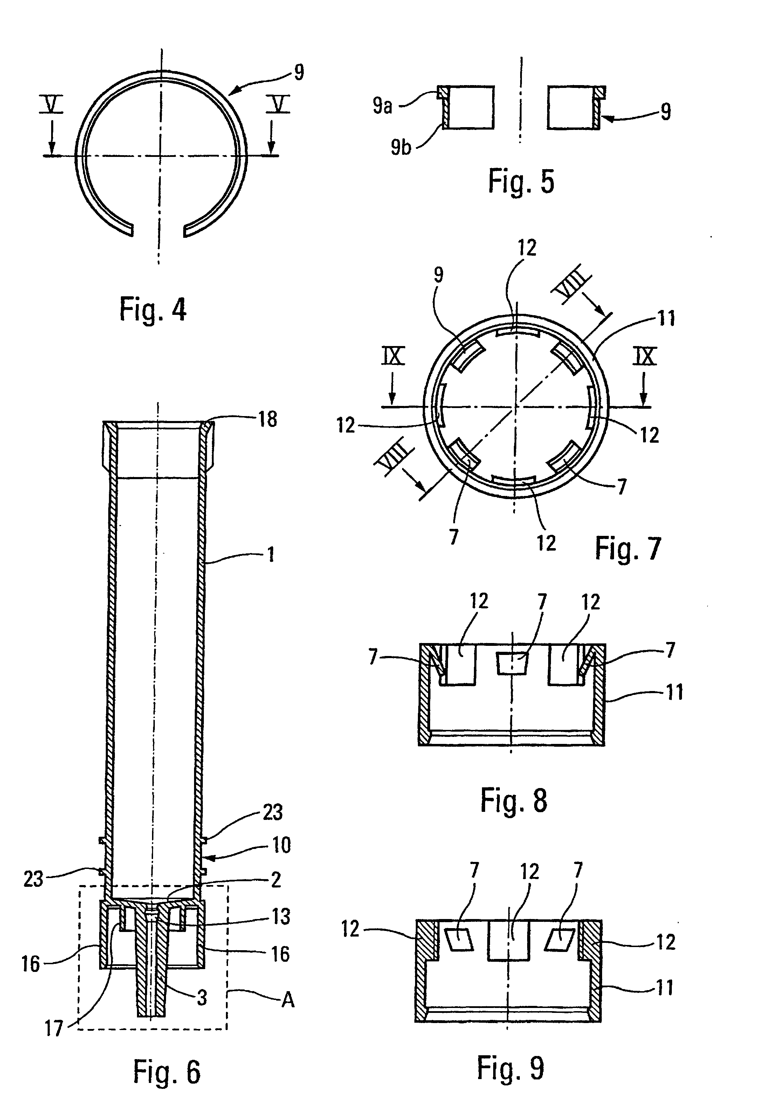

Comme mieux illustré à la figure 6, la portion 18 du corps 1 coopérant avec les ergots souples 7, est constituée de l'extrémité ouverte du corps 1 de forme tronconique, s'évasant vers l'extrémité ouverte du corps 1.

Cette portion 18 pourrait également être formée d'un ou plusieurs épaulements formés en surépaisseur sur la périphérie du corps 1.

Avantageusement, les ergots souples 7 sont réalisés d'un seul tenant avec le fourreau par exemple dans une matière plastique ou autre. Naturellement, le fourreau lui-même peut être réalisé en une seule partie ou plusieurs parties assemblées.

Conformément à l'invention, la seringue comporte un circlips 9 monté autour du corps 1 dans une position comprimée entre le fourreau 5 et le corps 1 dans la première position de blocage.

Dans une seconde position de blocage, illustrée sur la partie droite de la figure 3, le circlips 9 est dans une position relâchée et comporte au moins une partie 9a de contour extérieur de dimension supérieure à la dimension intérieure du fourreau 5.

Ainsi, le circlips 9 permet de bloquer le corps 1 dans le fourreau 5 lorsqu'il est dans cette seconde position de blocage, dans un sens inverse du premier sens de blocage assuré par les ergots souples 7.

Dans cette seconde position de blocage, le ressort 6 est au moins partiellement débandé et l'aiguille 8 est rentrée dans le fourreau 5, le passage de la première position à la seconde position se faisant par la coopération du piston 4 avec le ou les ergots souples 7 pour libérer sensiblement en fin de course du piston 4 l'ergot 7 de ladite position 18 du corps 1 et provoquer ainsi, sous l'effet du ressort 6, le recul du corps 1 dans le fourreau 5 jusqu'à son blocage en seconde position par le circlips 9.

Pour passer de la première position à la seconde position de blocage, le piston comporte une partie 22 susceptible de venir directement en contact avec les ergots souples 7, sensiblement en fin de course d'injection: cette partie peut être réalisée sous forme d'un élargissement de la tige de piston, ou d'une jupe portée par le piston.

Des moyens formant gorge 10 sont adaptés à loger le circlips 9.

Comme illustré à la figure 6, le corps 1 comporte dans cet exemple, deux épaulements circulaires 23, formés sur sa paroi cylindrique extérieure et définissant entre eux une gorge 10 adaptée à loger le circlips.

Bien entendu, ces épaulements circulaires pourraient être interrompus ou même être remplacés par des ergots ponctuels permettant de maintenir en place le circlips autour du corps 1, dans un plan transversal.

Les moyens formant gorge 10 sont situés de préférence à la base du corps 1. Ils peuvent également être situés à n'importe quel endroit du corps 1, y compris sur une jupe 16 prolongeant le corps 1 et dont la fonction est décrite plus loin dans la description.

Comme illustré aux figures 7, 8 et 9, le fourreau 5 comporte à une extrémité une partie formant manchon 11.

Cette partie formant manchon 11 peut former une seule pièce avec le reste du fourreau 5 ou bien être rapportée, par exemple par clipsage, autour de la paroi extérieure du fourreau 5.

Dans cet exemple, la partie formant manchon 11 constitue l'extrémité du fourreau, ce dernier ayant ainsi une partie allongée logeant le corps 1 dans la première position de blocage et une partie formant manchon 11 de diamètre intérieur supérieur à celui de la partie allongée du fourreau 5.

Comme illustré à la figure 3, le circlips 9 est logé à l'intérieur de la partie formant manchon 11, dans la seconde position de blocage, de sorte qu'il est protégé par celui-ci et rendu non-accessible à l'utilisateur.

Le ou les ergots souples 7 sont fixés à la partie formant manchon 11.

Ils sont au nombre de quatre dans cet exemple et sont disposés en quadrature sur la paroi intérieure de la partie formant manchon 11.

Les ergots souples 7 sont ainsi constitués de languettes flexibles s'étendant vers l'intérieur de la partie formant manchon 11.

Ces ergots 7 forment vers l'intérieur du fourreau 5 une saillie d'arrêt unidirectionnel dans un sens et une rampe dans l'autre.

Ainsi, le montage du corps 1 dans le fourreau 5 est facilité, les ergots souples 7 s'escamotant contre la paroi de la partie formant manchon 11 du fourreau 5 lors du passage de la partie 18 du corps 1.

Ils reprennent ensuite leur position en saillie pour bloquer la sortie du corps 1.

En outre, la partie formant manchon 11 comporte au moins un épaulement 12 adapté à coopérer en butée avec le circlips 9 dans la seconde position de blocage pour bloquer le corps 1 dans le fourreau 5 dans ledit premier sens de blocage.

Ces épaulements 12 sont également au nombre de quatre dans cet exemple de réalisation et disposés en quadrature sur la paroi intérieure de la partie formant manchon 11, entre les ergots souples 7.

Ces épaulements 12 sont formés de préférence de surépaisseurs s'étendant vers l'intérieur de la partie formant manchon, mais à une distance suffisamment faible de la paroi interne de la partie formant manchon 11 pour ne pas gêner le passage du corps 1 à l'intérieur de la partie formant manchon 11.

Les ergots 7 et les épaulements 12 prévus à l'intérieur de la partie formant manchon 11 peuvent être réalisés d'une seule pièce avec celle-ci, par exemple par moulage.

Comme illustré aux figures 4 et 5, le circlips 9 a une section transversale en forme de L, la jambe 9b de la forme en L étant logée entre le fourreau 5 et le corps 1 dans la seconde position de blocage.

Dans la première position de blocage, la jambe 9b de la forme en L est logée dans les moyens formant gorge 10 autour du corps 1, le circlips 9 étant maintenu comprimé par les parois intérieures du fourreau 5 en contact avec le pied 9a de la forme en L du circlips.

Dans la seconde position de blocage, la jambe 9b de la forme en L est maintenue dans les moyens formant gorge 10 et vient en contact avec la paroi intérieure du fourreau 5, dans sa partie allongée.

Le pied 9a de la forme en L du circlips s'étend dans la partie formant manchon 11 et empêche toute réintroduction du corps 1 dans le fourreau 5. Le pied 9a de la forme en L du circlips viendrait en effet en butée contre l'extrémité de la partie allongée du fourreau 5 si le corps 1 est repoussé vers l'intérieur du fourreau 5.

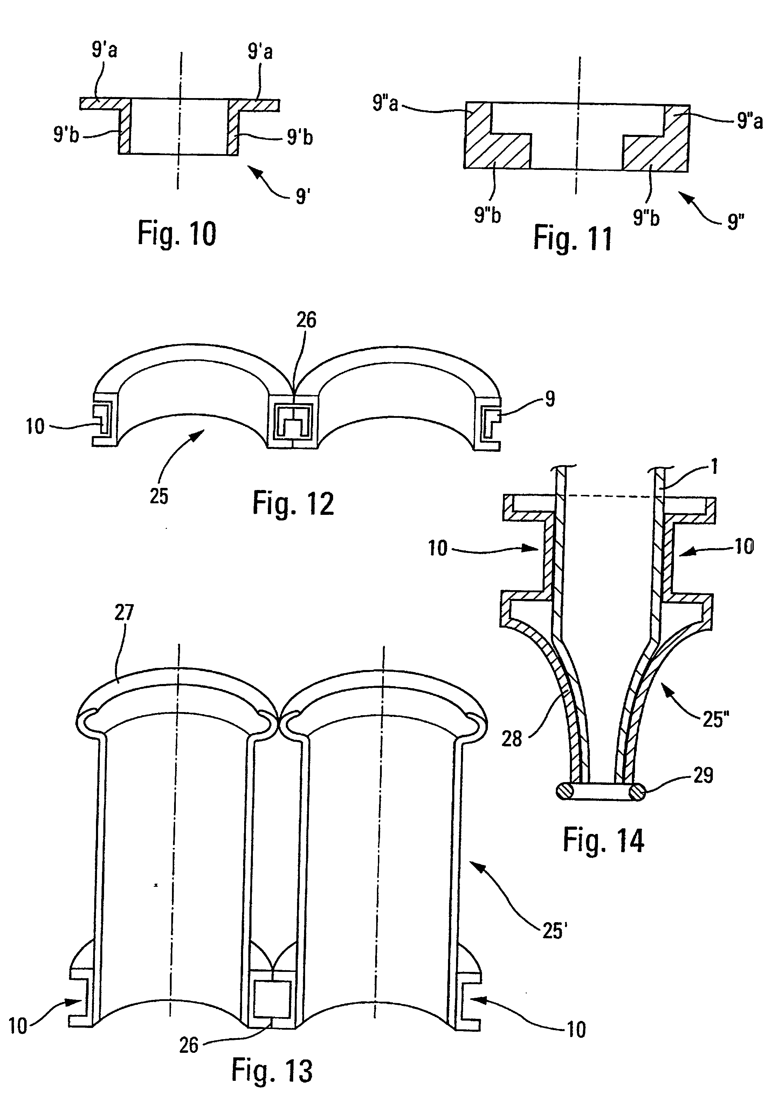

Comme mieux illustré à la figure 10, l'extrémité 2 du corps 1 portant les moyens de support d'aiguille 3 comporte un orifice 13 débouchant dans les moyens de support d'aiguille 3.

Cet orifice 13 relie le corps 1 à l'alésage 20 prévu pour alimenter l'aiguille d'injection en substance médicamenteuse.

Une extrémité 14 du piston 4 comporte un téton 15 adapté à se loger en force dans l'orifice 13, sensiblement en fin de course du piston 4.

L'orifice 13 est ainsi bouché par le téton 13, le piston 4 restant solidaire au fond du corps 1.

Le téton 15 est de préférence fixé à l'extrémité 14 du piston 4 par une zone amincie, de sorte que, si l'utilisateur cherche à retirer le piston 4 du corps 1, le téton 15 restera prisonnier de l'orifice 13 et se détachera de l'extrémité 14 du piston 4.

L'alésage 20 sera donc également obturé par le téton 15, empêchant d'utiliser la seringue pour une nouvelle injection.

Dans cet exemple, le téton 15 est formé de deux troncs de cône solidaires par leur base, l'orifice 13 ayant une forme complémentaire.

L'extrémité 2 du corps 1 comporte en outre deux jupes annulaires 16, 17, s'étendant autour des moyens de support d'aiguille d'injection 3, et définissant un logement annulaire pour recevoir le ressort 6.

Le fonctionnement de la seringue va maintenant être décrit en référence aux figures 1, 2 et 3.

En position de stockage, illustrée à la figure 1, les ergots souples 7 coopèrent avec le corps 1 pour effectuer un premier blocage du corps 1 dans le fourreau 5 correspondant à l'armement de la seringue, où le ressort 6 est comprimé. Les mêmes ergots 7 sont susceptibles de s'écarter sous l'action de la jupe 22 quant le piston 4 est enfoncé dans le corps 1. Le ressort 6 se détend alors et repousse le fourreau 5 du corps 1 jusqu'à la seconde position de blocage dans laquelle les épaulements 12 coopèrent avec le circlips 9 (position montrée sur la figure 3).

Les épaulements 12 et le circlips empêchent donc de retirer le corps 1 et son aiguille 8 du fourreau 5 qui la recouvre alors.

Le circlips 9 permet en outre d'interdire une nouvelle sortie de l'aiguille 8 hors du fourreau. De la sorte, après l'injection, le corps 1 reste bloqué, aussi bien en avant qu'en arrière, dans la position représentée à la droite de la figure 3 et l'aiguille 8 est inaccessible.

En outre, le piston 4 reste bloqué au fond du corps 1, en bout de course, grâce au téton 15 retenu dans l'orifice 13 de l'extrémité 2 du corps 1.

Bien entendu, l'invention n'est pas limitée à l'exemple décrit ci-dessus et de nombreuses modifications peuvent être apportées à celui-ci sans sortir du cadre de l'invention.

A la figure 5, le circlips de blocage de forme semi-annulaire et de section transversale en forme de L, comporte un pied 9a constituant la partie du circlips 9 coopérant avec un épaulement du fourreau pour empêcher la réintroduction du corps 1 dans ce dernier et bloquer le coulissement du corps 1 dans ce fourreau.

Comme illustré aux figures 10 et 11, la taille de la jambe 9b et du pied 9a du circlips en L peut varier. Ainsi, à la figure 10, le pied 9'a et la jambe 9'b du circlips ont des longueurs et épaisseurs identiques.

A la figure 11, et contrairement au circlips illustré à la figure 5, le pied 9''a du circlips a une épaisseur inférieure à l'épaisseur de la jambe 9''b du circlips 9''.

Par ailleurs, les figures 12 à 14 illustrent différents moyens de réaliser la gorge 10 sur le corps 7 de la seringue.

Ainsi, au lieu de former cette gorge 10 par l'ajout des épaulements circulaires 23 formant butées surmoulées sur le corps 1 comme illustré à la figure 6, un dispositif indépendant 25, 25' ou 25'' peut être fixé sur le corps 1 de la seringue.

Un tel dispositif permet d'équiper des seringues en verre, éventuellement du type seringues préremplies, ou même des seringues en plastique traditionnelles.

A la figure 12, le dispositif 25 est formé d'un anneau de section transversale sensiblement en forme de U qui forme ainsi la gorge 10. Cet anneau peut être constitué de deux demi-anneaux reliés par une charnière 26. Il suffit alors de pivoter les demi-anneaux sur la charnière 26 pour refermer l'anneau 25 sur le corps 1 d'une seringue. Cet anneau 25 peut éventuellement être solidarisé au corps 1 par collage ou soudage.

Selon un autre principe illustré à la figure 13, le dispositif 25' peut avoir une forme sensiblement cylindrique équipée sur son pourtour d'une gorge 10 semblable à celle décrite à la figure 12.

Le dispositif 25' comporte deux demi-cylindres montés en pivotement sur une charnière 26, de manière à être refermée autour du corps 1 d'une seringue comme dans le dispositif de la figure 12.

En outre, le cylindre 25' comporte à une extrémité une gorge annulaire 27 ouverte vers l'intérieur du cylindre 25' et adaptée à épouser une forme complémentaire circulaire du corps de seringue afin de fixer le dispositif 25' sur le corps 1 et d'empêcher son coulissement dans la direction longitudinale du corps.

De même, à la figure 14, un dispositif 25'' comporte un anneau équipé d'une gorge 10 semblable à celle décrite à la figure 12 et prolongé d'un manchon 28 sensiblement tronconique adapté à rentrer en force sur l'extrémité du corps 1 de seringue portant l'aiguille d'injection.

Cette extrémité du corps de seringue peut comporter une excroissance 29 adaptée à bloquer en translation le dispositif 25'' une fois celui-ci monté sur le corps de seringue.

Ces dispositifs 25, 25' et 25'' permettent ainsi de fournir facilement une gorge 10 adaptée à loger un circlips de blocage 9 sur le corps 1 d'une seringue traditionnelle.

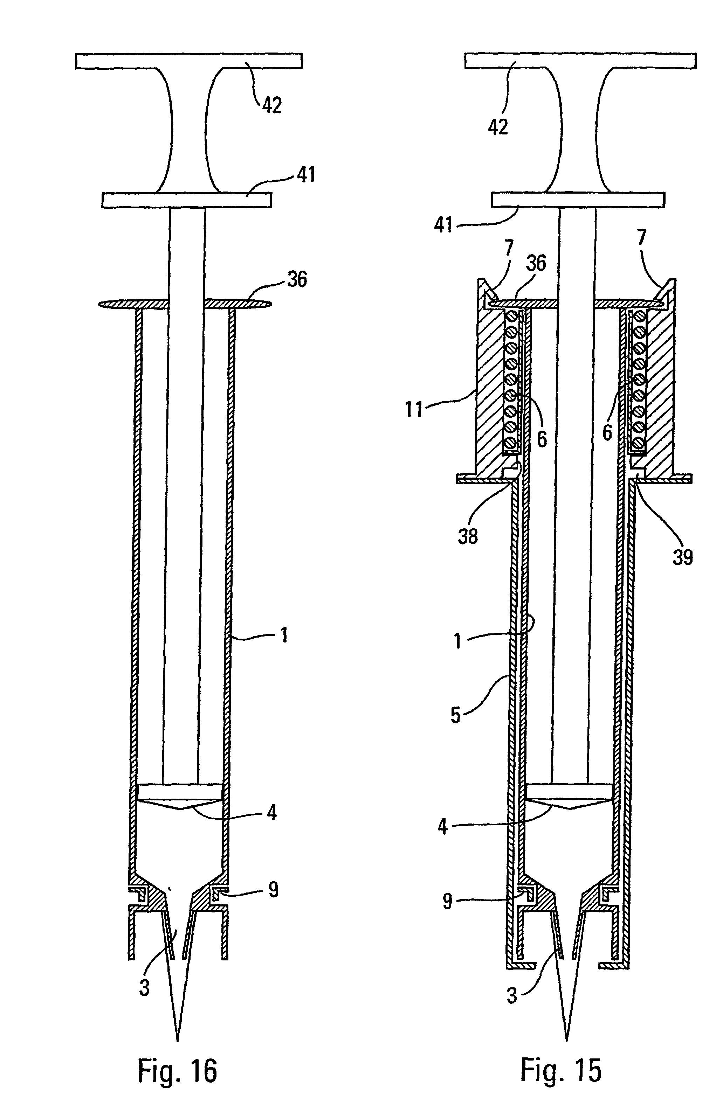

La seringue représentée sur les figures 15 à 18 comprend, comme celle représentée sur les figures 1 à 3, un corps 1 portant à l'une de ses extrémités, des moyens de support 3 pour une aiguille d'injection, un piston 4 mobile à l'intérieur du corps 1, un fourreau de protection 5 à l'intérieur duquel est susceptible de coulisser le corps 1.

A son extrémité opposée à l'aiguille, le fourreau 5 porte un manchon 11 dont le diamètre est élargi par rapport à celui du fourreau 5.

L'extrémité libre du manchon 11 porte des ergots 7 qui en position de stockage de la seringue (voir figure 15) bloquent la collerette 36 portée par l'extrémité adjacente du corps 1 par rapport au manchon 11.

La réalisation selon les figures 15 à 18 diffère de celle des figures 1 à 3 principalement par la disposition du ressort de compression 6 entre le manchon 11 et la partie du corps 1 adjacente à sa collerette d'extrémité 36.

Les extrémités opposées du ressort 6 s'appuient respectivement contre la collerette 36 du corps et un épaulement intérieur 38 du manchon 11 faisant saillie vers le corps 1.

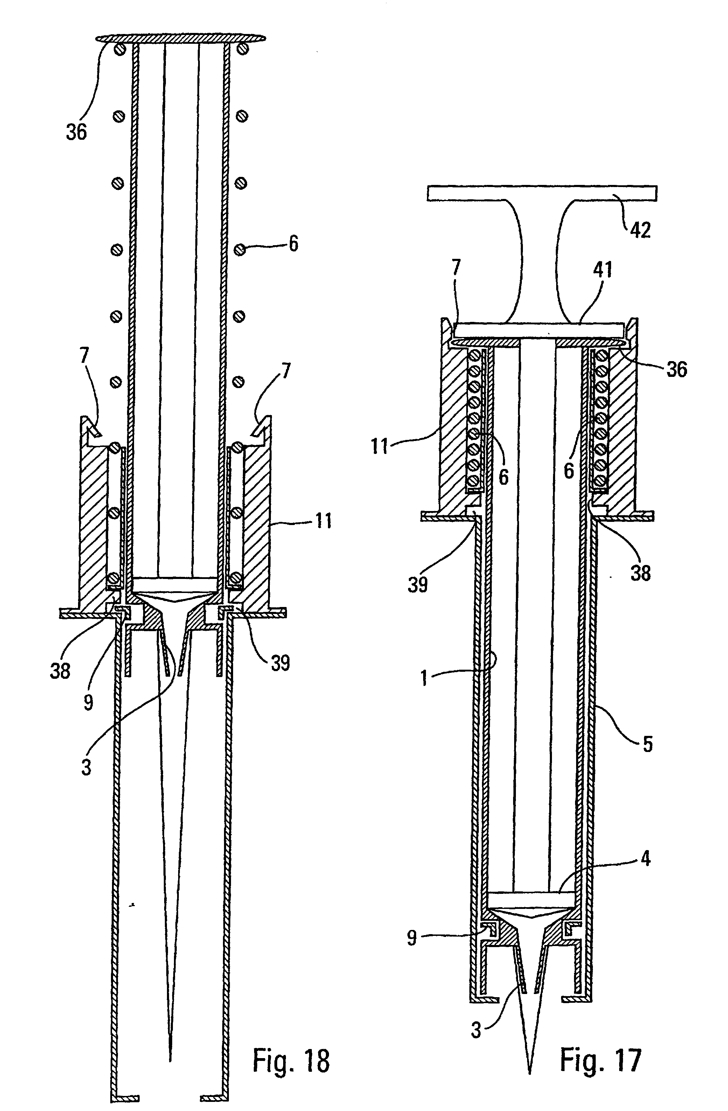

Entre l'épaulement 38 du manchon 11 et l'extrémité adjacente du fourreau 5 est prévue une gorge 39 dans laquelle peut se relâcher élastiquement, dans ladite seconde position de blocage, un circlips 9 engagé sur l'extrémité du corps 1 adjacente à l'aiguille pour bloquer le corps 1 par rapport au fourreau 5 après déplacement du corps vers l'extérieur jusqu'à la position montrée par la figure 18.

La seringue représentée sur les figures 15 à 18 fonctionne de la façon suivante :

En position de stockage, comme indiqué sur la figure 15, les ergots élastiques 7 bloquent le corps 1 par rapport au fourreau 1, le ressort 6 étant dans une position comprimée.

Les ergots élastiques 7 sont repoussés vers l'extérieur (voir figure 17) par la collerette 41 prévue sous la poignée 42 du piston 4, lorsque ce dernier est enfoncé dans le corps 1.

De ce fait, la collerette 36 du corps 1 est libérée par rapport au manchon 11 et le ressort 6 peut alors se détendre pour déplacer le corps 1 par rapport au fourreau 5 jusqu'à ce que le circlips 9 s'engage dans la gorge 39, comme montré sur la figure 18.

Dans cette position, on ne peut retirer le corps 1 du fourreau 5 et l'aiguille est entièrement contenue à l'intérieur du fourreau 5.

De même, dans la position représentée sur la figure 18, il n'est pas possible de déplacer le corps 1 pour réaliser une nouvelle sortie de l'aiguille hors du fourreau 5.

## Revendications

1. Seringue à aiguille auto-escamotable, comprenant un corps (1) portant à une extrémité (2) des moyens de support d'aiguille d'injection (3), un piston (4) mobile dans ledit corps (1), un fourreau (5) à l'intérieur duquel est susceptible de coulisser ledit corps (1), le fourreau (5) comportant à une extrémité une partie formant manchon (11), un ressort (6) disposé entre le fourreau (5) et le corps (1), au moins un ergot souple (7) coopérant en butée avec une portion (18) dudit corps (1) pour définir une première position de blocage du corps (1) par rapport au fourreau (5) dans un premier sens, le ressort (6) étant comprimé et l'aiguille (8) sortie du fourreau (5) dans cette première position, et des moyens de blocage pour bloquer le corps (1) dans le fourreau (5) dans une seconde position de blocage, le ressort (6) étant au moins partiellement débandé et l'aiguille (8) étant rentrée dans le fourreau (5) dans cette seconde position, le passage de la première position à la seconde position se faisant par la coopération du piston (4) avec ledit ergot souple (7) pour libérer sensiblement en fin de course du piston (4) l'ergot (7) de ladite portion (18) du corps (1) et provoquer ainsi, sous l'effet du ressort (6), le recul du corps (1) dans le fourreau (5) jusqu'à son blocage en seconde position, **caractérisée en ce que** les moyens de blocage comprennent un circlips (9) monté autour dudit corps (1) dans une position comprimée entre le fourreau (5) et le corps (1) dans ladite première position de blocage et dans une position relâchée dans ladite seconde position de blocage, le circlips (9) ayant au moins une partie (9a) logée à l'intérieur de la partie formant manchon (11) dans ladite seconde position de blocage entre ladite extrémité du fourreau et au moins un épaulement (12) de la partie formant manchon (11) adapté à coopérer en butée avec le circlips (9) dans ladite seconde position de blocage pour bloquer le corps (1) dans le fourreau (5) dans ledit premier sens.

2. Seringue conforme à la revendication 1, **caractérisée en ce que** le circlips (9) a une section transversale en forme de L, la jambe (9b) de la forme en L étant logée entre le fourreau (5) et le corps (1) dans ladite seconde position de blocage.

3. Seringue conforme à l'une des revendications 1 ou 2, **caractérisée en ce que** ledit corps (1) comprend des moyens formant gorge (10) adaptés à loger ledit circlips (9).

4. Seringue conforme à l'une des revendications 1 à 3, **caractérisée en ce que** ledit ergot souple (7) est fixé à la partie formant manchon (11).

5. Seringue conforme à l'une des revendications 1 à 4, **caractérisée en ce que** l'extrémité (2) du corps (1) portant les moyens de support d'aiguille (3) comporte un orifice (13) débouchant dans les moyens de support d'aiguille (3), une extrémité (14) du piston (4) comportant un téton (15) adapté à se loger en force dans ledit orifice (13).

6. Seringue conforme à la revendication 5, **caractérisée en ce que** ledit téton (15) est fixé à l'extrémité (14) du piston (4) par une zone amincie (15a).

7. Seringue conforme à l'une des revendications 1 à 6, **caractérisée en ce que** le circlips est de forme semi-annulaire et de section transversale en forme de L, le pied (9a) de la forme en L constituant ladite partie (9a) adaptée à coopérer avec un épaulement de la partie formant manchon (11) pour bloquer le coulissement du corps (1) dans ledit fourreau (5).

8. Seringue conforme à l'une des revendications 1 à 7, **caractérisée en ce que** ledit manchon (11) du fourreau a un diamètre élargi par rapport à celui du fourreau (5), l'extrémité libre de ce manchon (11) portant des ergots (7) qui bloquent une partie (36) portée par le corps, lorsque la seringue est en position de stockage.

9. Seringue conforme à la revendication 8, **caractérisée en ce que** ledit ressort (6) est disposé entre le manchon (11) et le corps (1), ce ressort (6) étant comprimé entre la partie (36) du corps (1) et un épaulement intérieur (38) du manchon (11).

10. Seringue conforme à l'une des revendications 8 ou 9, **caractérisée en ce que** le manchon (11) comporte une gorge (39) dans laquelle peut se relâcher élastiquement un circlips (9) engagé sur l'extrémité du corps (1) adjacente à l'aiguille pour bloquer ce corps (1) dans ladite seconde position de blocage après déplacement de ce dernier vers l'extérieur du fourreau (5).

## Patentansprüche

1. Spritze mit selbst einziehbarer Nadel, welche einen Körper (1) umfasst, der an einem Endpunkt (2) Stützvorrichtungen für eine Injektionsnadel (3) aufweist, einen in dem Körper (1) beweglichen Kolben (4), eine Hülse (5), deren Innenseite geeignet ist, dass der Körper (1) darin gleiten kann, wobei die Hülse (5) an einem Endpunkt einen eine Muffe (11) bildenden Bereich aufweist, eine zwischen der Hülse (5) und dem Körper (1) angeordnete Feder (6), wenigstens einen biegbaren Vorsprung (7), der mit einem Bereich (18) des Körpers (1) als Anschlag zusammenwirkt, um eine erste Blockierstellung des Körpers (1) in Bezug zur Hülse (5) in eine erste Richtung zu definieren, wobei in dieser ersten Stellung die Feder (6) zusammengedrückt ist und die Nadel (8) aus der Hülse (5) herauskommt, und Blockiervorrichtungen, um den Körper (1) in der Hülse (5) in einer zweiten Position zu blockieren, wobei die Feder (6) wenigstens teilweise entspannt und die Nadel (8) in dieser zweiten Stellung in die Hülse (5) zurückgezogen ist, wobei der Übergang von der ersten Stellung zu der zweiten Stellung durch das Zusammenwirken des Kolbens (4) mit dem biegbaren Vorsprung (7) entsteht, um am Hubende des Kolbens (4) den Vorsprung (7) des Bereichs (18) des Körpers (1) freizugeben, und danach unter der Wirkung der Feder (6) das Zurückspringen des Körpers (1) aus der Hülse (5) bis zu der Blockierung in der zweiten Stellung auszulösen, **dadurch gekennzeichnet, dass** die Blockiervorrichtungen einen um den Körper (1) angebrachten Sicherungsring (9) umfassen, in einer zwischen der Hülse (5) und dem Körper (1) zusammengedrückten Position in der ersten Blockierstellung und in einer gelockerten Position in der zweiten Blockierstellung, wobei der Sicherungsring (9) wenigstens einen Abschnitt (9a) aufweist, angeordnet an der Innenseite des die Muffe (11) bildenden Bereichs in der zweiten Blockierposition zwischen dem Endpunkt der Hülse und wenigstens dem Absatz (12) des die Muffe (11) bildenden Bereichs, angepasst, um als Anschlag mit dem Sicherungsring (9) in der zweiten Blockierstellung zusammenzuwirken, um den Körper (1) in der Hülse (5) in der ersten Richtung zu blockieren.

2. Spritze gemäß Anspruch 1, **dadurch gekennzeichnet, dass** der Sicherungsring (9) einen querliegenden Abschnitt in Form eines "L" aufweist, wobei der Schenkel (9b) des "L" angeordnet ist zwischen der Hülse (5) und dem Körper (1) in der zweiten Blockierstellung.

3. Spritze gemäß einem der Ansprüche 1 oder 2, **dadurch gekennzeichnet, dass** der Körper (1) Vorrichtungen aufweist, die eine Kehle (10) bilden, angepasst zum Aufnehmen des Sicherungsrings (9).

4. Spritze gemäß irgendeinem der Ansprüche 1 bis 3, **dadurch gekennzeichnet, dass** der biegbare Vorsprung (7) an dem die Muffe (11) bildenden Bereich befestigt ist.

5. Spritze gemäß irgendeinem der Ansprüche 1 bis 4, **dadurch gekennzeichnet, dass** der die Vorrichtungen zum Stützen der Nadel (3) aufweisende Endpunkt (2) des Körpers (1) eine in die Vorrichtung zum Stützen der Nadel (3) mündende Öffnung (13) beinhaltet, einen Endpunkt (14) des Kolbens (4), der einen Ansatz (15) beinhaltet, angepasst, um sich mit Kraft in die Öffnung (13) zu führen.

6. Spritze gemäß Anspruch 5, **dadurch gekennzeichnet, dass** der Ansatz (15) an dem Endpunkt (14) des Kolbens (4) durch einen verengten Bereich befestigt ist.

7. Spritze gemäß irgendeinem der Ansprüche 1 bis 6, **dadurch gekennzeichnet, dass** der Sicherungsring halbringförmig ausgebildet ist und einen querliegenden Abschnitt in Form eines "L" aufweist, wobei der Fuß (9a) der "L"-Form, der diesen Abschnitt (9a) bildet, angepasst ist, um mit einem Absatz des die Muffe (11) bildenden Bereichs zum Blockieren des Gleitens des Körpers (1) in der Hülse (5) zusammenzuwirken.

8. Spritze gemäß irgendeinem der Ansprüche 1 bis 7, **dadurch gekennzeichnet, dass** die Muffe (11) der Hülse (5) einen größeren Durchmesser hat als die Hülse (5), der freie Endpunkt der Muffe (11) Vorsprünge (7) aufweist, die einen in dem Körper enthaltenen Abschnitt (36) blockieren, wenn die Spritze in Lagerposition ist.

9. Spritze gemäß Anspruch 8, **dadurch gekennzeichnet, dass** die Feder (6) zwischen der Muffe (11) und dem Körper (1) angeordnet ist, wobei die Feder (6) zwischen dem Abschnitt (36) des Körpers (1) und einem inneren Absatz (38) der Muffe (11) zusammengedrückt ist.

10. Spritze gemäß einem der Ansprüche 8 oder 9, **dadurch gekennzeichnet, dass** die Muffe (11) eine Kehle (39) aufweist, in welcher sich ein Sicherungsring (9) elastisch lockern kann, verbunden mit dem Endpunkt des Körpers, der an die Nadel angrenzt, um den Körper (1) in der zweiten Blockierposition nach Verlagerung der Nadel in Richtung Außenseite der Hülse (5) zu blockieren.

## Claims

1. A syringe with a self-retracting needle, comprising a body (1) bearing at one end (2) means (3) for supporting an injection needle; a piston (4) which is movable in said body (1); a sheath (5) inside which said body (I) can slide, the sheath (5) having at one end a portion in the form of a sleeve (11); a spring (6) disposed between the sheath (5) and the body (1); at least one flexible lug (7) cooperating with a portion (18) of said body (1) to form a limit stop for defining a first position for immobilizing the body (1) in relation to the sheath (5) in a first direction, the spring (6) being compressed and the needle (8) emerging from the sheath (5) in this first position; and immobilizing means for immobilizing the body (1) in the sheath (5) in a second immobilizing position, the spring (6) being at least partly relaxed and the needle (8) having re-entered the sheath (5) in this second position, the change from the first position to the second position being effected by cooperation of the piston (4) with said flexible lug (7) to release the lug (7) from said portion (18) of the body (1), more or less at the end of the stroke of the piston (4), thus causing, under the influence of the spring (6), the body (1) to retract into the sheath (5) until it is immobilized in the second position, **characterized in that** the immobilizing means comprise a circlip (9) fitted around said body (1) in a position compressed between the sheath (5) and the body (1) in said first immobilizing position and in a relaxed position in said second immobilizing position, the circlip (9) having at least one part (9a) lodged inside the sleeve portion (11), in said second immobilizing position, between said end of the sheath and at least one shoulder (12) of the sleeve (11) which is designed to cooperate with the circlip (9) to form a limit stop in said second immobilizing position so as to immobilize the body (1) in the sheath (5) in said first direction.

2. A syringe according to Claim 1, **characterized in that** the circlip (9) has an L-shaped cross-section, the leg (9b) of the L shape being lodged between the sheath (5) and the body (1) in said second immobilizing position.

3. A syringe according to one of Claims 1 or 2, **characterized in that** said body (1) includes means taking the form of a groove (10) designed to accommodate said circlip (9).

4. A syringe according to one of Claims 1 to 3, **characterized in that** said flexible lug (7) is fixed to the sleeve portion (11).

5. A syringe according to one of Claims 1 to 4, **characterized in that** the end (2) of the body (1) bearing the needle-supporting means (3) has an orifice (13) opening into the needle-supporting means (3), one end (14) of the piston (4) having a nipple (15) designed to become lodged in said orifice (13) when force is applied.

6. A syringe according to Claim 5, **characterized in that** said nipple (15) is fixed to the end (14) of the piston (4) in a thinned-down area (15a).

7. A syringe according to one of Claims 1 to 6, **characterized in that** the circlip is semi-annular in shape and has an L-shaped cross-section, the foot (9a) of the L shape forming said part (9a) designed to cooperate with a shoulder of the sleeve portion (11) to prevent the body (1) from sliding in said sheath (5).

8. A syringe according to one of Claims 1 to 7, **characterized in that** said sleeve (11) of the sheath has an enlarged diameter compared with that of the sheath (5), the free end of this sleeve (11) bearing lugs (7) which immobilize a part (36) carried by the body (1), when the syringe is in its storage position.

9. A syringe according to Claim 8, **characterized in that** said spring (6) is disposed between the sleeve (11) and the body (1), this spring (6) being compressed between the part (36) of the body (1) and an inner shoulder (38) of the sleeve (11).

10. A syringe according to one of Claims 8 or 9, **characterized in that** the sleeve (11) has a groove (39) into which a circlip (9), engaged on the end of the body (1) adjacent to the needle, can elastically relax to immobilize this body (1) in said second immobilizing position after outward displacement of the latter from the sheath (5).
